# EUROPEAN PATENT SPECIFICATION

(11) **EP 3 075 724 B1**
(45) Date of publication and mention of the grant of the patent: **12.07.2023**
(21) Application number: 15162055.6
(22) Date of filing: 31.03.2015
(51) Int. Cl.: C07C 233/18

(54) **SOLID FORM OF AGOMELATINE**
FESTE FORM VON AGOMELATIN
FORME SOLIDE DE L'AGOMÉLATINE

(43) Date of publication of application: 05.10.2016
(73) Proprietor: F.I.S.- Fabbrica Italiana Sintetici S.p.A., 36075 Montecchio Maggiore (VI) (IT)
(72) Inventor: BYDLINSKI, GREGORY, Kirkland , Québec H9J 3A4 (CA); GAGNONI, ALESSANDRO, 20016 Luino (IT); GERMANI, ANTONIO, 6900 Lugano (CH)
(74) Representative: Leganza, Alessandro

(56) References cited:
- WO-A1-2012/168665

## Description

### Field of the Invention

The present invention relates to a new solid form of N-[2-(7-methoxynaphthalen-1-yl)ethyl]acetamide of formula (I) 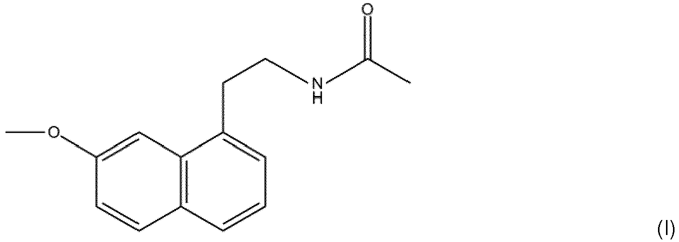 with pyruvic acid of formula (II) 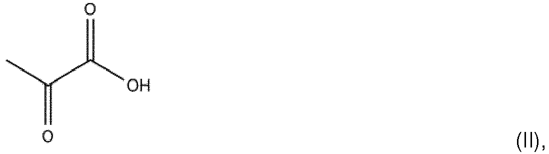 processes for its preparation and pharmaceutical compositions containing it.

### Background of the Invention

The drug compound N-[2-(7-methoxynaphthalen-1-yl)ethyl]acetamide, also known by its International Nonproprietary Name (INN) agomelatine, is an agonist of the melatonin receptors MT₁ and MT₂ and an antagonist of the serotonin receptor 5-HT_{2C}. It is marketed under the trade names Valdoxan , Thymanax^{®} and Melitor^{®} for the treatment of major depression in adult patients.

Agomelatine, its preparation and its use have been disclosed in EP 0 447 285.

Although agomelatine is freely soluble (> 100 mg/ml) in various organic solvents (e.g. ethanol, methanol, methylene chloride), it is practically insoluble in purified water (< 0.1 mg/ml). Therefore, a solid form of agomelatine with improved dissolution properties in a physiological (i.e. aqueous) environment for use in an immediate release pharmaceutical formulation would be advantageous.

The property of some solid compounds, like agomelatine, to occur in different crystal forms is called polymorphism. A particular molecule may give rise to a variety of polymorphs with distinct crystal structures that result in distinct physical and physicochemical properties. Some of these differences in physical properties have been widely used to distinguish polymorphic forms by assessing thermal behaviours (measured by, e.g., thermogravimetric analysis (TGA) or differential scanning calorimetry (DSC)), powder X-ray diffraction patterns (PXRD), infrared absorption, Raman fingerprints, and solid state nuclear magnetic resonance (NMR) spectra. Others, like different hygroscopicities, solubilities, and mechanical properties (e.g. flowability) can affect the stability, bioavailability and processability of an active pharmaceutical compound and give thus reasons to search for yet unknown polymorphs.

Numerous amorphous and crystal forms of agomelatine have been described (e.g. WO 2007/015002, WO 2007/015003, WO 2007/015004, WO 2009/095555, WO 2010/102554, WO 2011/006387, WO 2012/093402, WO 2012/126385, WO 2012/126386, WO 2012/130837, CN 101955440, CN 102001959, CN 102030673). However, form II as disclosed in WO 2005/077887 appears to be the thermodynamically most stable form. All the other crystalline forms of agomelatine described in the above mentioned publications reveal a more or less pronounced disposition to convert into form II and were thus found to be not reproducible or physically unstable during storage. The physical instability also applies to form I, which is the crystal form that results from the preparation of agomelatine described in EP 0 447 285 (Tinant et al, Acta Cryst 1994, C50(6), 907-10).

Several attempts have been made to establish stable solid forms of agomelatine by combining it with one or more additional compounds in molecular complexes. WO 2012/146371, for example, describes cocrystals of agomelatine with citric acid, maleic acid and benzenesulfonic acid. WO 2012/046253 describes cocrystals of agomelatine with oxalic acid. WO 2012/168665 describes cocrystals of agomelatine with parahydroxybenzoic acid, citric acid, oxalic acid, gallic acid, maleic acid, malonic acid, glutaric acid, glycolic acid and ketoglutaric acid. Zheng et al., Cryst Growth Des 2011, 11(2), 466-71, describe cocrystals of agomelatine with acetic acid and ethylene glycol. Yan et al., Cryst Growth Des 2012, 12(5), 2226-31, describe cocrystals of agomelatine with urea, glycolic acid, isonicotinamide and methyl 4-hydroxybenzoate.

Still there remains a demand for additional cocrystals of agomelatine of sufficient purity and superior physical and chemical stability that can be used for the preparation of pharmaceutical compositions.

Cocrystals are generally defined as homogeneous crystalline structures comprising two or more components that can be atoms or molecules in a definite stoichiometric ratio. Contrary to salts, where the arrangement in the crystal lattice is based on ion pairing, the components of a cocrystal structure interact via non-ionic and also non-covalent weak intermolecular interactions such as hydrogen bonding, van der Waals forces and π-interactions. The term "cocrystal" as used in this application includes solvates, where not all of the components of a cocrystal exist as individual solids at ambient conditions, and hydrates, where water is among the components of a cocrystal. In other words, solvates and hydrates are considered as subgroups of the general concept of cocrystals.

### Summary of the Invention

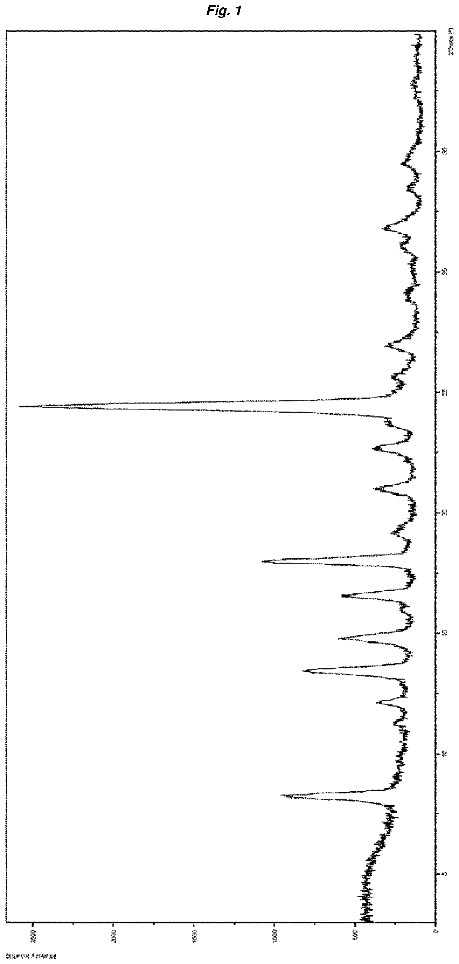

In a first aspect, this invention provides a pyruvic acid cocrystal of agomelatine. The crystalline form of this novel cocrystal can be characterized by a PXRD pattern having peaks at 8.27°, 13.46°, 16.58°, 17.99° and a double peak at 24.23°/24.43°2θ ± 0.2°2Θ. The crystalline form of the cocrystals of agomelatine and pyruvic acid can be further characterized by a PXRD pattern having one or more additional peak(s) selected from the peaks at 14.72°/14.86°, 20.99°, 22.65° and 31.8°2θ ± 0.2°2θ. Furthermore, the crystalline form of the cocrystals of agomelatine and pyruvic acid can be characterized by a PXRD pattern substantially as depicted in Figure 1.

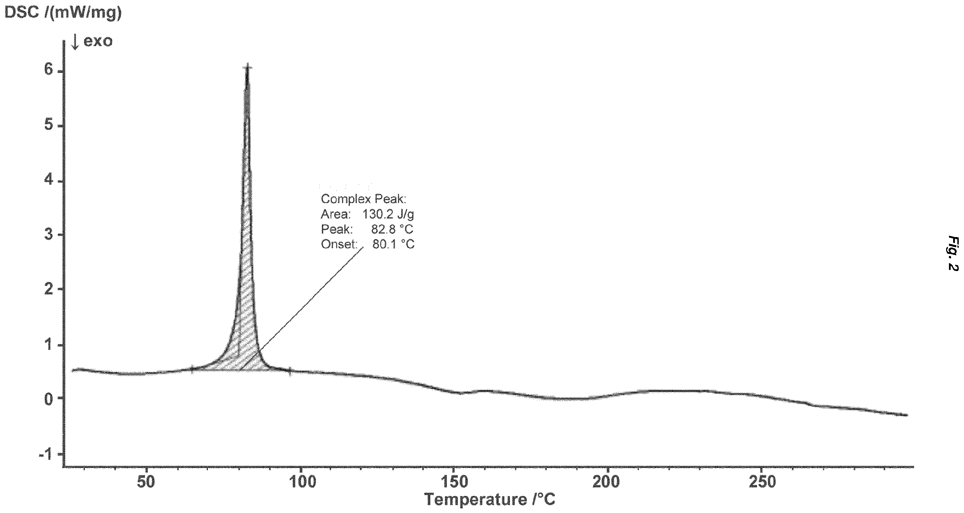

Alternatively, the crystalline form of the cocrystals of agomelatine and pyruvic acid can be characterized by a DSC profile having an endothermic peak at 82.8 °C ± 1.5 °C with an onset at 80.1 °C ± 1.5 °C. Furthermore, the crystalline form of the cocrystals of agomelatine and pyruvic acid can be characterized by a DSC profile substantially as depicted in Figure 2.

In another aspect, this application discloses methods for the preparation of the novel agomelatine pyruvic acid cocrystals by either dissolving agomelatine directly in pyruvic acid or by adding pyruvic acid to a solution of agomelatine in a polar organic solvent, followed by the addition of a suitable anti-solvent and isolation of the precipitated agomelatine pyruvic acid cocrystals.

In a further aspect, this invention provides a pharmaceutical composition comprising agomelatine pyruvic acid cocrystals, and at least one pharmaceutically acceptable excipient, and a process for the preparation of the pharmaceutical composition by combining the agomelatine pyruvic acid cocrystals, and at least one pharmaceutically acceptable excipient.

### Brief Description of the Drawings

**Figure 1** shows a PXRD pattern of agomelatine pyruvic acid cocrystals obtained according to the procedure of Example 1.
**Figure 2** shows a DSC profile of agomelatine pyruvic acid cocrystals obtained according to the procedure of Example 1.
**Figure 3** shows a diagram of the average values of Table 1. It shows the kinetic dissolution in aqueous medium of agomelatine pyruvic acid cocrystal in comparison to agomelatine form I.

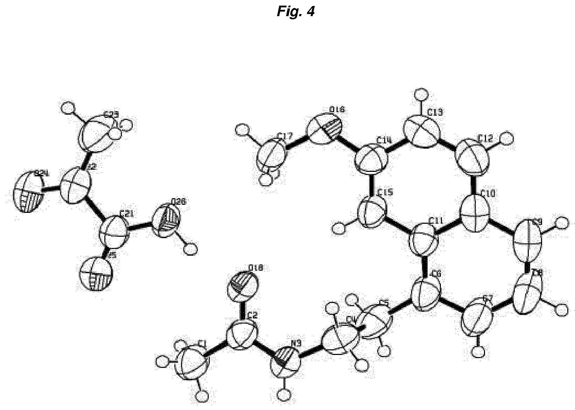

**Figure 4** shows the structure of agomelatine pyruvic acid cocrystal with labels and ellipsoids at the 50 % probability.

### Detailed Description of the Invention

A first aspect of the invention relates to a new solid form of agomelatine of formula (I) 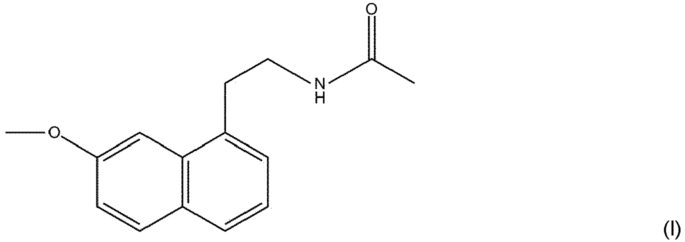 with pyruvic acid of formula (II)

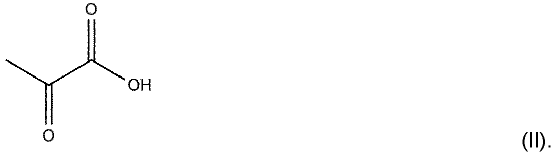

In particular this invention relates to substantially pure cocrystals of agomelatine and pyruvic acid. These Cocrystals are homogeneous crystalline structures of agomelatine and pyruvic acid in a definite stoichiometric ratio. This stoichiometric ratio is 1 : 1.

The crystalline form of the cocrystals of agomelatine and pyruvic acid can be characterized by a PXRD pattern having peaks at 8.27°, 13.46°, 16.58°, 17.99° and a double peak at 24.23°/24.43° 2θ ± 0.2° 2Θ. The crystalline form of the cocrystals of agomelatine and pyruvic acid can be further characterized by a PXRD pattern having one or more additional peak(s) selected from the peaks at 14.72714.86°, 20.99°, 22.65° and 31.8° 2Θ ± 0.2° 2Θ. Furthermore, the crystalline form of the cocrystals of agomelatine and pyruvic acid can be characterized by a PXRD pattern substantially as depicted in Figure 1. The skilled person will understand that such graphical representations are generally influenced by factors such as variations in sample preparation and purity and variations in instrument response, which may result in small variations of peak intensities and peak positions. Nevertheless, the person skilled in the art would be readily capable of evaluating whether two sets of data are identifying the same crystal form or two different forms by comparing the graphical data disclosed herein with graphical data generated for a comparison sample. Therefore, the term "substantially as depicted in Figure 1" includes crystalline forms characterized by graphical data with small variations well known to the skilled person.

Alternatively, the crystalline form of the cocrystals of agomelatine and pyruvic acid can be characterized by a DSC profile having an endothermic peak at 82.8 °C ± 1.5 °C with an onset at 80.1 °C ± 1.5 °C. Furthermore, the crystalline form of the cocrystals of agomelatine and pyruvic acid can be characterized by a DSC profile substantially as depicted in Figure 2. The skilled person will understand that such graphical representations are generally influenced by factors such as variations in sample preparation and purity and variations in instrument response, which may result in small variations of peak intensities and peak positions. Nevertheless, the person skilled in the art would be readily capable of evaluating whether two sets of data are identifying the same crystal form or two different forms by comparing the graphical data disclosed herein with graphical data generated for a comparison sample. Therefore, the term "substantially as depicted in Figure 2" includes crystalline forms characterized by graphical data with small variations well known to the skilled person.

Alternatively, the crystalline form of the cocrystals of agomelatine and pyruvic acid can be characterized by a single crystal X-ray structure of a monoclinic system in space group I2/a with approximately the following cell parameters at 293 K: a = 15.102(09) Å, b = 15.698(26) Å, c = 15.120(15) Å, β = 105.34(5)°, V = 3456.(83) Å³.

The agomelatine pyruvic acid cocrystals can be prepared by dissolving agomelatine in pyruvic acid, adding the anti-solvent water, and isolating the precipitated agomelatine pyruvic acid cocrystals. Alternatively, the agomelatine pyruvic acid cocrystals can be prepared by adding pyruvic acid to a solution of agomelatine in a polar organic solvent, ethyl acetate, adding an anti-solvent, being a nonpolar organic solvent, or a liquid hydrocarbon, or heptane, and isolating the precipitated agomelatine pyruvic acid cocrystals.

The agomelatine pyruvic acid cocrystals according to the present invention show superior stability and solubility. Agomelatine pyruvic acid cocrystals are therefore particularly suitable for the use in pharmaceutical technology. Accordingly, this invention further encompasses a pharmaceutical composition comprising agomelatine pyruvic acid cocrystals, as described above, and at least one pharmaceutically acceptable excipient, and a process for the preparation of the pharmaceutical composition by combining the agomelatine pyruvic acid cocrystals, as described above, and at least one pharmaceutically acceptable excipient. The agomelatine pyruvic acid cocrystals and the pharmaceutical composition containing them can be used as a medicament, for example for the treatment of depression.

### Examples

### Powder X-ray diffractometry

Measurements were performed on an X'Pert PRO PANalytical diffractometer equipped with a PW3373/00 Cu LFF DK184511 X-ray tube and an X'Celerator RTMS (Real Time Multiple Strip) detector under the following conditions:

| | |
|---|---|
| Measurement type: | Single scan |
| Sample mode: | Reflection |

### Scan

| | |
|---|---|
| Scan axis: | Gonio |
| Scan range: | 3.0010 - 39.9997°2θ |
| Step size: | 0.0167°2θ |
| Counting time: | 12.7 s/step |
| No. of points: | 2214 |
| Scan mode: | Continuous |

### Used wavelength

| | |
|---|---|
| Voltage: | 40 kV |
| Current: | 40 mA |
| Intended wavelength type: | CuK_{α1} |
| K_{α1}: | 1 .540598 Å |
| K_{α2}: | 1.544426 Å |
| K_{α2}/Kα₁ intensity ratio: | 0.50 |
| K_{α}: | 1.541874 Å |
| K_{β}: | 1.392250 Å |

### Incident beam path

| | |
|---|---|
| Radius: | 240.0 mm |

### DSC analysis

DSC analysis was performed with a Netzsch DSC 200 F3 Maia^{®}. A sample of 2.38 mg of agomelatine pyruvic acid cocrystals according to Example 1 was weighed into a closed alumina crucible and was heated at 10 °C/min from 30 to 350 °C.

The agomelatine pyruvic acid cocrystal according to the present invention is characterized by a melting point corresponding to a sharp endothermic peak at about 83 °C with an onset at about 80 °C.

### Example 1: Preparation of agomelatine pyruvic acid cocrystals

1 g (4.11 mmol) of agomelatine was charged in a 50-mL round bottom flask, equipped with a magnetic stirring bar. 5 mL of pyruvic acid were transferred into the reaction flask and the mixture was stirred at room temperature until total dissolution of the starting material (approx. 5 minutes). 20 mL of H₂O were added to the clear solution obtained. The formation of a slightly gray solid was immediately observed and the stirring was maintained for 48 hours in order to obtain a finer powder. After that the precipitate was recovered under vacuum, washed three times with 10 mL H₂O and dried at 40 °C for 72 hours.

1.25 g of off-white solid were recovered (Y = 91.9 %, purity (HPLC) > 99.8 %).

### Example 2: Preparation of agomelatine pyruvic acid cocrystals with ethyl acetate

1 g (4.11 mmol) of agomelatine was dissolved in 10 ml of ethyl acetate at 50 °C. Approximately 5 eq of pyruvic acid was added to the solution and the mixture was stirred at room temperature for 6 hours. To the clear solution obtained, 60 ml of heptane was added under stirring. The resulting suspension was stirred further for 24 hours at room temperature and the off-white precipitate was recovered under vacuum, washed with heptane (35 ml) and dried at 35 °C under vacuum for 18 hours.

1.26 g of off-white solid were recovered (Y = 92.6 %, purity (HPLC) > 99.7 %).

### Example 3: Stability tests

Agomelatine pyruvic acid cocrystal according to Example 1 was subjected to stability tests for seven days at 25 °C, 60 % RH and 40 °C, 75 % RH, respectively. The samples showed excellent chemical stability as determined by HPLC.

### Example 4: Kinetic dissolution tests

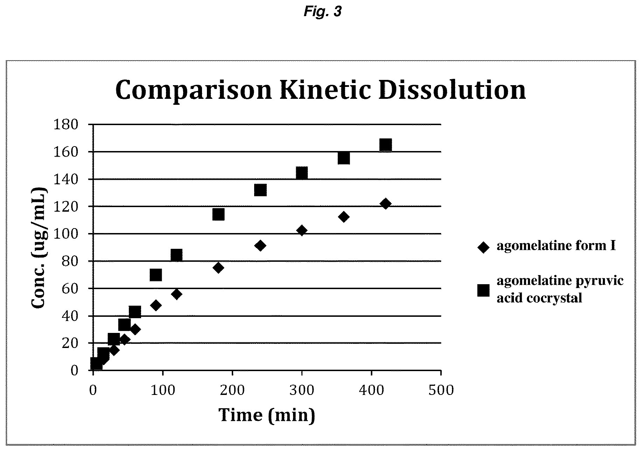

The tests were performed in duplicate on 13 mm tablets containing 200 mg agomelatine (form I) or agomelatine pyruvic acid cocrystal, which were prepared on a Digital Hydraulic Press at a force of about 8 metric tons. The tablets were placed in 100 mL of saline solution in a paddle apparatus at 100 rpm and 37 °C ± 0.5 °C. A 3 mL sample was withdrawn from each vessel at fixed time points, filtered through a 0.45 µm filter (discarding the first 1 mL), analyzed by HPLC, and compared to a previously prepared reference standard calibration curve. Immediately after each sampling, the solutions in the vessels were replenished with 3 mL of saline. Table 1 below and Figure 3 show the superior solubility in aqueous medium of the agomelatine pyruvic acid cocrystal as compared to agomelatine form I.

**Table 1:**

| Time [min] | Form I [µg/mL] | | | Pyruvic Acid Cocrystal [µg/mL] | | |
|---|---|---|---|---|---|---|
| | #1 | #2 | ∅ | #1 | #2 | ∅ |
| 5 | 3.39 | 4.38 | 3.89 | 5.21 | 5.45 | 5.33 |
| 15 | 8.20 | 9.32 | 8.76 | 12.55 | 13.42 | 12.99 |
| 30 | 14.80 | 15.58 | 15.19 | 22.75 | 24.10 | 23.43 |
| 45 | 22.55 | 23.81 | 23.18 | 33.41 | 37.03 | 35.22 |
| 60 | 30.05 | 31.53 | 30.79 | 42.71 | 50.65 | 46.68 |
| 90 | 47.49 | 50.84 | 49.17 | 70.03 | 77.31 | 73.67 |
| 120 | 55.73 | 58.72 | 57.23 | 84.65 | 90.24 | 87.45 |
| 180 | 75.07 | 74.85 | 74.96 | 114.37 | 114.58 | 114.48 |
| 240 | 91.32 | 86.85 | 89.09 | 131.99 | 131.80 | 131.9 |
| 300 | 102.54 | 96.76 | 99.65 | 144.64 | 144.74 | 144.69 |
| 360 | 112.42 | 106.36 | 109.39 | 155.52 | 156.49 | 156.01 |
| 420 | 121.98 | 114.14 | 118.06 | 165.30 | 163.14 | 164.22 |

## Claims

1. Agomelatine pyruvic acid cocrystal wherein the stoichiometric ratio is 1 : 1.

2. The agomelatine pyruvic acid cocrystal according to claim 1, **characterized by** a single crystal X-ray structure of a monoclinic system in space group I2/a with approximately the following cell parameters at 293 K: a = 15.102(09) Å, b = 15.698(26) Å, c = 15.120(15) Å, β = 105.34(5)°, V = 3456.(83) Å³.

3. The agomelatine pyruvic acid cocrystal according to any one of claims 1 or 2 **characterized by** a powder X-ray diffraction (PXRD) pattern having a peak at 24.4° 2θ ± 0.2° 2θ.

4. The agomelatine pyruvic acid cocrystal according to claim 3 **characterized by** a PXRD pattern having one or more additional peak(s) selected from the peaks at 8.3°, 13.5° and 18° 2θ ± 0.2° 2θ.

5. The agomelatine pyruvic acid cocrystal according to claim 4 **characterized by** a PXRD pattern having one or more additional peak(s) selected from the peaks at 14.9°, 16.6°, 21°, 22.7° and 31.8° 2θ ± 0.2° 2θ.

6. The agomelatine pyruvic acid cocrystal according to any one of claims 1 or 2 **characterized by** a PXRD pattern substantially as depicted in Figure 1.

7. The agomelatine pyruvic acid cocrystal according to any of the preceding claims **characterized by** a Differential Scanning Calorimetry (DSC) profile having an endothermic peak at 82.8 °C ± 1.5 °C with an onset at 80.1 °C ± 1.5 °C.

8. The agomelatine pyruvic acid cocrystal according to any one of claims 1 to 6 **characterized by** DSC profile substantially as depicted in Figure 2.

9. A process for the preparation of the agomelatine pyruvic acid cocrystal according to any of the preceding claims comprising the steps of
a) dissolving agomelatine in pyruvic acid;
b) adding an anti-solvent, wherein the anti-solvent is water; and
c) isolating the precipitated agomelatine pyruvic acid cocrystal.

10. A process for the preparation of the agomelatine pyruvic acid cocrystal according to any one of claims 1 to 8 comprising the steps of
a) providing a solution of agomelatine in a polar organic solvent, wherein the polar organic solvent is ethyl acetate;
b) adding pyruvic acid to the solution obtained in step a);
c) adding an anti-solvent, wherein the anti-solvent is a nonpolar organic solvent, or liquid hydrocarbon, or heptane; and
d) isolating the precipitated agomelatine pyruvic acid cocrystal.

11. A pharmaceutical composition comprising the agomelatine pyruvic acid cocrystal according to any one of claims 1 to 8, and at least one pharmaceutically acceptable excipient.

12. A process for the preparation of a pharmaceutical composition comprising combining the agomelatine pyruvic acid cocrystal according to any one of claims 1 to 8, and at least one pharmaceutically acceptable excipient.

13. The agomelatine pyruvic acid cocrystal according to any one of claims 1 to 8 or the pharmaceutical composition according to claim 11 for use as a medicament.

14. The agomelatine pyruvic acid cocrystal according to any one of claims 1 to 8 or the pharmaceutical composition according to claim 11 for use in the treatment of depression.

## Patentansprüche

1. Agomelatin-Brenztraubensäure-Cokristall, wobei das stöchiometrische Verhältnis 1:1 ist.

2. Agomelatin-Brenztraubensäure-Cokristall nach Anspruch 1, **gekennzeichnet durch** eine Einkristall-Röntgenstruktur eines monoklinen Systems in der Raumgruppe I2/a mit annähernd den folgenden Zellparametern bei 293 K: a = 15,102(09) Å, b = 15,698(26) Å, c = 15,120(15) Å, β = 105,34(5)°, V = 3456,(83) Å³.

3. Agomelatin-Brenztraubensäure-Cokristall nach einem der Ansprüche 1 oder 2, **gekennzeichnet durch** ein Pulverröntgenbeugungs- (PXRD)-Spektrum mit einem Peak bei 24,4° 2Θ ± 0,2° 2Θ.

4. Agomelatin-Brenztraubensäure-Cokristall nach Anspruch 3, **gekennzeichnet durch** ein PXRD-Spektrum mit einem oder mehreren zusätzlichen Peak(s) ausgewählt aus den Peaks bei 8,3°, 13,5° und 18° 2θ ± 0,2° 2Θ.

5. Agomelatin-Brenztraubensäure-Cokristall nach Anspruch 4, **gekennzeichnet durch** ein PXRD-Spektrum mit einem oder mehreren zusätzlichen Peak(s) ausgewählt aus den Peaks bei 14,9°, 16,6°, 21°, 22,7° und 31,8° 2θ ± 0,2° 2θ.

6. Agomelatin-Brenztraubensäure-Cokristall nach einem der Ansprüche 1 oder 2, **gekennzeichnet durch** ein PXRD-Spektrum, das im Wesentlichen wie in Figur 1 abgebildet ist.

7. Agomelatin-Brenztraubensäure-Cokristall nach einem der vorhergehenden Ansprüche, **gekennzeichnet durch** ein Differentialscanningkalorimetrie- (DSC)-Profil mit einem endothermen Peak bei 82,8 °C ± 1,5 °C mit einem Beginn bei 80,1 °C ± 1,5 °C.

8. Agomelatin-Brenztraubensäure-Cokristall nach einem der Ansprüche 1 bis 6, **gekennzeichnet durch** ein DSC-Profil, das im Wesentlichen wie in Figur 2 abgebildet ist.

9. Verfahren zur Herstellung des Agomelatin-Brenztraubensäure-Cokristalls nach einem der vorhergehenden Ansprüche, umfassend die Schritte:
a) Lösen von Agomelatin in Brenztraubensäure;
b) Zugeben eines Antilösungsmittels, wobei das Antilösungsmittel Wasser ist; und
c) Isolieren des ausgefällten Agomelatin-Brenztraubensäure-Cokristalls.

10. Verfahren zur Herstellung des Agomelatin-Brenztraubensäure-Cokristalls nach einem der Ansprüche 1 bis 8, umfassend die Schritte:
a) Bereitstellen einer Lösung von Agomelatin in einem polaren organischen Lösungsmittel, wobei das polare organische Lösungsmittel Ethylacetat ist;
b) Zugeben von Brenztraubensäure zu der in Schritt a) erhaltenen Lösung;
c) Zugeben eines Antilösungsmittels, wobei das Antilösungsmittel ein unpolares organisches Lösungsmittel oder flüssiger Kohlenwasserstoff oder Heptan ist; und
d) Isolieren des ausgefällten Agomelatin-Brenztraubensäure-Cokristalls.

11. Pharmazeutische Zusammensetzung, umfassend den Agomelatin-Brenztraubensäure-Cokristall nach einem der Ansprüche 1 bis 8 und mindestens einen pharmazeutisch annehmbaren Hilfsstoff.

12. Verfahren zur Herstellung einer pharmazeutischen Zusammensetzung, umfassend Kombinieren des Agomelatin-Brenztraubensäure-Cokristalls nach einem der Ansprüche 1 bis 8 und mindestens eines pharmazeutisch annehmbaren Hilfsstoffs.

13. Agomelatin-Brenztraubensäure-Cokristall nach einem der Ansprüche 1 bis 8 oder pharmazeutische Zusammensetzung nach Anspruch 11 zur Verwendung als Medikament.

14. Agomelatin-Brenztraubensäure-Cokristall nach einem der Ansprüche 1 bis 8 oder pharmazeutische Zusammensetzung nach Anspruch 11 zur Verwendung in der Behandlung von Depression.

## Revendications

1. Co-cristal d'acide pyruvique d'agomélatine dans lequel le ratio stoechiométrique est 1 : 1.

2. Co-cristal d'acide pyruvique d'agomélatine selon la revendication 1, **caractérisé par** une structure monocristalline à rayons X d'un système monoclinique dans le groupe spatial I2/a avec approximativement les paramètres cellulaires suivants à 293 K : a = 15,102(09) Å, b = 15,698(26) Å, c = 15,120(15) Å, β = 105,34(5)°, V = 3456,(83) Å³.

3. Co-cristal d'acide pyruvique d'agomélatine selon l'une quelconque des revendications 1 ou 2, **caractérisé par** un schéma de diffraction aux rayons X ayant un pic à 24,4° 2Θ ± 0,2° 2Θ.

4. Co-cristal d'acide pyruvique d'agomélatine selon la revendication 3, **caractérisé par** un schéma de diffraction aux rayons X ayant un ou plusieurs pic(s) supplémentaire(s) sélectionné(s) parmi les pics à 8,3°, 13,5° et 18° 2θ ± 0,2° 2θ.

5. Co-cristal d'acide pyruvique d'agomélatine selon la revendication 4, **caractérisé par** un schéma de diffraction aux rayons X ayant un ou plusieurs pic(s) supplémentaire(s) sélectionné(s) parmi les pics à 14,9°, 16,6°, 21°, 22,7° et 31,8° 2θ ± 0,2° 2θ.

6. Co-cristal d'acide pyruvique d'agomélatine selon l'une quelconque des revendications 1 ou 2, **caractérisé par** un schéma de diffraction aux rayons X substantiellement tel qu'illustré à la figure 1.

7. Co-cristal d'acide pyruvique d'agomélatine selon l'une quelconque des revendications précédentes, **caractérisé par** un profil d'analyse calorimétrique différentielle (ACD) ayant un pic endothermique à 82,8 °C ± 1,5 °C avec une survenue à 80,1 °C ± 1,5 °C.

8. Co-cristal d'acide pyruvique d'agomélatine selon l'une quelconque des revendications 1 à 6, **caractérisé par** un profil ACD substantiellement tel qu'illustré à la figure 2.

9. Procédé pour la préparation du co-cristal d'acide pyruvique d'agomélatine selon l'une quelconque des revendications précédentes, comprenant les étapes de
a) dissolution de l'acide pyruvique d'agomélatine ;
b) ajout d'un anti-solvant, dans lequel l'anti-solvant est l'eau ; et
c) isolement du co-cristal d'acide pyruvique d'agomélatine précipité.

10. Procédé pour la préparation du co-cristal d'acide pyruvique d'agomélatine selon l'une quelconque des revendications 1 à 8, comprenant les étapes de
a) fourniture d'une solution d'agomélatine dans un solvant organique polaire, dans lequel le solvant organique polaire est l'acétate d'éthyle ;
b) ajout de l'acide pyruvique à la solution obtenue à l'étape a) ;
c) ajout d'un anti-solvant, dans lequel l'anti-solvant est un solvant organique apolaire ou un hydrocarbure liquide ou l'heptane ; et
d) isolement du co-cristal d'acide pyruvique d'agomélatine précipité.

11. Composition pharmaceutique comprenant le co-cristal d'acide pyruvique d'agomélatine selon l'une quelconque des revendications 1 à 8, et au moins un excipient pharmaceutiquement acceptable.

12. Procédé pour la préparation d'une composition pharmaceutique comprenant la combinaison du co-cristal d'acide pyruvique d'agomélatine selon l'une quelconque des revendications 1 à 8, et au moins un excipient pharmaceutiquement acceptable.

13. Co-cristal d'acide pyruvique d'agomélatine selon l'une quelconque des revendications 1 à 8 ou composition pharmaceutique selon la revendication 11 pour une utilisation comme médicament.

14. Co-cristal d'acide pyruvique d'agomélatine selon l'une quelconque des revendications 1 à 8 ou composition pharmaceutique selon la revendication 11 pour une utilisation dans le traitement de la dépression.
